# EUROPÄISCHE PATENTANMELDUNG

(11) **EP 4 733 250 A1**
(43) Veröffentlichungstag der Anmeldung: **29.04.2026**
(21) Anmeldenummer: 24020322.4
(22) Anmeldetag: 22.10.2024
(51) Int. Cl.: C01B 3/38, C07C 4/02, C10G 1/06, C10G 3/00, C10G 45/26, F25J 3/02

(54) **VERFAHREN UND ANLAGE ZUR ERZEUGUNG EINES ODER MEHRERER VERFAHRENSPRODUKTE**

(71) Anmelder: Linde GmbH, 82049 Pullach (DE)
(72) Erfinder: Ettner, Florian, 82049 Pullach (DE); Köhler, Regina, 82049 Pullach (DE)
(74) Vertreter: Fischer, Werner

(57) **Zusammenfassung**

Ein Verfahren zur Verfahren (100) zur Erzeugung eines oder mehrerer Verfahrensprodukte wird vorgeschlagen, das ein Bereitstellen eines flüssigen Hydrierungseinsatzes (101) mit ungesättigten Kohlenwasserstoffen mit drei und mehr Kohlenstoffatomen, ein Hydrieren (11) des Hydrierungseinsatzes (101) oder eines Teils hiervon Zustand unter Erhalt eines Hydrierungsprodukts (103), ein Bilden (12, 13) eines Reformierungseinsatzes (105) unter Verwendung des Hydrierungsprodukts (103) oder eines Teils hiervon, und ein Reformieren (14) des Reformierungseinsatzes (105) unter Erhalt eines Reformierungsprodukts (107) umfasst. Eine entsprechende Anlage wird ebenfalls vorgeschlagen.

## Beschreibung

### Gebiet

Die vorliegende Offenbarung betrifft ein Verfahren und eine Anlage zur Erzeugung eines oder mehrerer Verfahrensprodukte.

### Hintergrund

Verfahren und Vorrichtungen zum Dampfspalten oder Steamcracken (engl. Steam Cracking) von Kohlenwasserstoffen sind bekannt und z.B. im Artikel "Ethylene" in Ullmann's Encyclopedia of Industrial Chemistry, online seit 15. April 2007, DOI 10.1002/14356007.a10_045.pub2, beschrieben.

Beim Steamcracken werden sogenannte Spaltgase erhalten, bei denen es sich um Kohlenwasserstoffgemische mit Kohlenwasserstoffen unterschiedlicher Kettenlänge, Sättigung und Struktur handelt. Um aus einem Spaltgas die erwünschten Produkte zu gewinnen, muss dieses getrennt werden. Aus dem Stand der Technik sind hierzu unterschiedliche Trennsequenzen bekannt und beispielsweise in dem erwähnten Artikel ausführlich beschrieben. Diese können beispielsweise eine sogenannte Demethanisierung gefolgt von einer Deethanisierung, oder eine Deethanisierung gefolgt von einer Demethanisierung, umfassen.

Typischerweise können in entsprechenden Trennsequenzen Kohlenwasserstoffgemische erhalten werden, die ein- und mehrfach ungesättigte Kohlenwasserstoffe mit drei und mehr Kohlenstoffatomen enthalten. Diese können nicht immer direkt verwertet werden, so dass eine Umsetzung zu anderen Produkten wünschenswert ist. Die vorliegende Offenbarung erstreckt sich dabei ausdrücklich auch auf die Nutzung entsprechender Kohlenwasserstoffe und Kohlenwasserstoffgemische aus anderen Quellen als dem Steamcracken.

Es gilt, Verfahren und Anlagen bereitzustellen, die eine Nutzung der genannten Kohlenwasserstoffe in effizienter Weise ermöglichen und bestimmte, nachfolgend noch im Detail erläuterte Nachteile bekannter Verfahren überwinden.

### Übersicht

Vor diesem Hintergrund werden ein Verfahren und eine Anlage zur Erzeugung eines oder mehrerer Verfahrensprodukte mit den Merkmalen der unabhängigen Patentansprüche vorgeschlagen. Ausgestaltungen sind Gegenstand der abhängigen Patentansprüche und der nachfolgenden Beschreibung.

Das vorgeschlagene Verfahren zur Erzeugung eines oder mehrerer Verfahrensprodukte umfasst ein Bereitstellen eines flüssigen Hydrierungseinsatzes mit ungesättigten Kohlenwasserstoffen mit drei und mehr Kohlenstoffatomen, ein Hydrieren des Hydrierungseinsatzes oder eines Teils hiervon in flüssigem Zustand unter Erhalt eines Hydrierungsprodukts, ein Bilden eines Reformierungseinsatzes unter Verwendung des Hydrierungsprodukts oder eines Teils hiervon, und ein Reformieren des Reformierungseinsatzes unter Erhalt eines Reformierungsprodukts.

Das vorgeschlagene Verfahren ermöglicht eine Verwendung von ungesättigten Kohlenwasserstoffen mit drei und mehr Kohlenstoffatomen, beispielsweise in entsprechenden Fraktionen aus Steamcrackern oder Ethylenanlagen, in Reformierungsverfahren, beispielsweise der Dampf- und/oder Autothermreformierung. Hierdurch werden Probleme, wie sie in Kombination mit anderen Verfahren auftreten können, beispielsweise ein erhöhter Sauerstoffbedarf in der Partialoxidation, vermieden. Gleichfalls reduzieren sich, wie unten ausführlich erläutert, Wärme- und Exothermieprobleme, die herkömmlicherweise nur aufwendig bewältigbar sind.

Die vorgeschlagene Verschaltung einer Flüssighydrierung einer Fraktion mit ungesättigten Kohlenwasserstoffen mit drei und mehr Kohlenstoffatomen mit einer Reformierung Hydrierungsprodukts ermöglicht also längerkettige ungesättigte Kohlenwasserstoffe als Einsatzstoff für reformerbasierte Anlagen zu nutzen, ohne dass aufwendige Maßnahmen zur Kühlung und Kontrolle der Exothermie wie in einer konventionellen Hydrierung oder Entschwefelung erforderlich sind. Außerdem wird die Gefahr der Rußbildung oder Polymerisation, wie sie bei Anwärmung längerkettiger ungesättigter Kohlenwasserstoffe zu erwarten ist, umgangen. Zu weiteren Erläuterungen sei auf die detaillierte Beschreibung der hier vorgeschlagenen Ausführungsformen im Zusammenhang mit den Figuren verwiesen.

In bestimmten Ausgestaltungen des vorgeschlagenen Verfahrens ist vorgesehen, dass das Bereitstellen des flüssigen Hydrierungseinsatzes das Steamcracken eines Kohlenwasserstoffgemischs unter Erhalt eines Spaltgases und das Bilden des Hydrierungseinsatzes unter Verwendung eines Teils des Spaltgases umfasst. Das Verfahren ermöglicht damit die vorteilhafte Verwertung entsprechender, beim Steamcracken anfallender Kohlenwasserstoffe.

In bestimmten Ausgestaltungen des vorgeschlagenen Verfahrens ist vorgesehen, dass das Bilden des Hydrierungseinsatzes unter Verwendung eines Teils des Spaltgases eine Deethanisierung umfasst. Diese kann einer Demethanisierung vor- oder nachgeschaltet sein. Eine schwere Fraktion aus der Deethanisierung kann als der Hydrierungseinsatz verwendet oder zu diesem aufbereitet werden.

In bestimmten Ausgestaltungen des vorgeschlagenen Verfahrens ist vorgesehen, dass das Verfahren eine Bereitstellung einer oder mehrerer Komponenten des Reformierungsprodukts als das Verfahrensprodukt oder eines der mehreren Verfahrensprodukte umfasst. Hierbei kann es sich beispielsweise um Wasserstoff oder Kohlenmonoxid handeln, die geeigneten Verwendungszwecken zuführbar sind.

In bestimmten Ausgestaltungen des vorgeschlagenen Verfahrens ist vorgesen, dass das Verfahren eine Umsetzung einer oder mehrerer Komponenten des Reformierungsprodukts zu dem Verfahrensprodukt oder einem der mehreren Verfahrensprodukte umfasst. Das vorgeschlagene Verfahren kann in diesem Fall als Verbundverfahren zur Bereitstellung unterschiedlichster Verfahrensprodukte dienen.

In bestimmten Ausgestaltungen des vorgeschlagenen Verfahrens ist vorgesehen, dass die soeben erwähnte Umsetzung eine Umsetzung von Wasserstoff mit Stickstoff zu Ammoniak und/oder von Kohlendioxid mit Wasserstoff zu Methanol umfasst.

In bestimmten Ausgestaltungen des vorgeschlagenen Verfahrens ist vorgesehen, dass die ungesättigten Kohlenwasserstoffe mit drei und mehr Kohlenstoffatomen mit bis zu 100 Gewichtsprozent in dem Hydrierungseinsatz enthalten sind. Das vorgeschlagene Verfahren und seine Ausgestaltungen ermöglichen damit eine stoffliche Verwertung auch derartiger Komponentengemische, die anderenfalls typischerweise rein thermisch verwertet werden.

In bestimmten Ausgestaltungen des vorgeschlagenen Verfahrens ist vorgesehen, dass die Kohlenwasserstoffe mit drei und mehr Kohlenstoffatomen Kohlenwasserstoffe mit bis zu 12 Kohlenstoffatomen umfassen. Auch entsprechend schwere Kohlenwasserstoffe können damit bearbeitet werden.

In bestimmten Ausgestaltungen des vorgeschlagenen Verfahrens ist vorgesehen, dass das Reformieren des Reformierungseinsatzes eine Dampf- und/oder Autothermreformierung umfasst. Auch entsprechende Kombinationen können verwendet werden, und die Wahl des jeweiligen Reformierungsverfahrens kann sich insbesondere nach den Produktanforderungen oder lokalen Gegebenheiten wie der Verfügbarkeit von Sauerstoff richten.

In bestimmten Ausgestaltungen des vorgeschlagenen Verfahrens ist vorgesehen, dass das Reformieren des Reformierungseinsatzes eine Vorreformierung umfasst. Auf diese Weise kann eine Bearbeitung der längerkettigen Verbindungen ohne Anpassung des zur Reformierung eingesetzten Katalysators und ohne Gefahr einer Verrußung oder Polymerisation erfolgen.

Die vorgeschlagene Anlage zur Erzeugung eines oder mehrerer Verfahrensprodukte ist zum Bereitstellen eines flüssigen Hydrierungseinsatzes mit ungesättigten Kohlenwasserstoffen mit drei und mehr Kohlenstoffatomen, zum Hydrieren des Hydrierungseinsatzes oder eines Teils hiervon in flüssigem Zustand unter Erhalt eines Hydrierungsprodukts, zum Bilden eines Reformierungseinsatzes unter Verwendung des Hydrierungsprodukts oder eines Teils hiervon, und zum Reformieren des Reformierungseinsatzes unter Erhalt eines Reformierungsprodukts eingerichtet.

Vorteile und Merkmale, die bezüglich des vorgeschlagenen Verfahrens und seinen Ausgestaltungen beschrieben wurden, gelten auch für die vorgeschlagene Anlage und umgekehrt. Diese werden entsprechend nur einmalig beschrieben, und auf die jeweiligen Erläuterungen kann verwiesen werden.

Entsprechendes gilt auch für eine Anlage, die dazu eingerichtet sein kann, ein Verfahren gemäß einer beliebigen Ausgestaltung durchzuführen.

### Zeichnungen

Im Rahmen der vorliegenden Offenbarung vorgeschlagene Aspekte werden anhand der beiliegenden Zeichnung näher erläutert. Dabei zeigen
Figur 1 ein nicht erfindungsgemäßes Verfahren;
Figur 2 ein Verfahren gemäß einer vorgeschlagenen Ausgestaltung; und
Figur 3 Verfahrensschritte zur Bereitstellung eines Hydrierungseinsatzes.

### Ausführungsformen

Die nachfolgend beschriebenen Ausführungsformen werden lediglich zu dem Zweck beschrieben, den Leser beim Verständnis der beanspruchten und zuvor erläuterten Merkmale zu unterstützen. Sie stellen lediglich repräsentative Beispiele dar und sollen hinsichtlich der Merkmale der Erfindung nicht abschließend und/oder beschränkend betrachtet werden. Es versteht sich, dass die zuvor und nachfolgend beschriebenen Vorteile, Ausführungsformen, Funktionen, Merkmale, Strukturen und/oder andere Aspekte nicht als Beschränkungen von Äquivalenten zu den Ansprüchen zu betrachten sind, und dass andere Ausführungsformen verwendet und Änderungen vorgenommen werden können, ohne vom Umfang der beanspruchten Erfindung abzuweichen.

Unterschiedliche Ausführungsformen der Erfindung können weitere zweckmäßige Kombinationen der beschriebenen Elemente, Komponenten, Merkmale, Teile, Schritte, Mittel usw. umfassen, aufweisen, aus ihnen bestehen oder im Wesentlichen aus diesen bestehen, auch wenn solche Kombinationen hier nicht spezifisch beschrieben sind. Darüber hinaus kann die Offenbarung andere Erfindungen umfassen, die gegenwärtig nicht beansprucht sind, die aber in Zukunft beansprucht werden können, insbesondere wenn sie vom Umfang der unabhängigen Ansprüche umfasst sind.

Erläuterungen, die sich auf Vorrichtungen, Apparate, Anordnungen, Systeme usw. gemäß Ausführungsformen der vorliegenden Erfindung beziehen, können auch für Verfahren, Prozesse, Methoden usw. gemäß den Ausführungsformen der vorliegenden Erfindung gelten und umgekehrt. Gleiche, gleich wirkende in ihrer Funktion einander entsprechende, baulich identische oder vergleichbar aufgebaute Elemente, Verfahrensschritte usw. können mit identischen Bezugszeichen angegeben sein.

Die nachfolgenden Erläuterungen und Definitionen, die einige Grundlagen der Erfindung betreffen, können für alle oder einen Teil der hier vorgestellten Ausgestaltungen gelten, und die Erläuterung bestimmter Aspekte im Zusammenhang mit nur einem Teil oder einer der Ausgestaltungen soll nicht dahingehend verstanden werden, dass diese Aspekte nicht auch mit anderen oder allen Ausgestaltungen, soweit technisch möglich und sinnvoll, verwirklicht sein können.

Sämtliche hier verwendete Prozentangaben können sich auf Mol-, Mengen- oder Volumenanteile beziehen. Druckangaben in bar sind, soweit nicht anders erläutert, insbesondere als Absolutdrücke zu verstehen.

Die Konjunktion "und/oder" soll, wenn in einer Aufzählung vor dem letzten Element der Aufzählung verwendet, so verstanden werden, dass alle in der Aufzählung zuvor genannten Begriffe in beliebiger Weise miteinander kombiniert werden können. Mit anderen Worten ist mit "A, B und/oder C" "A und/oder B und/oder C" oder "wenigstens eines der Elemente A, B, C in beliebiger Kombination" gemeint.

Die im Rahmen der vorliegenden Offenbarung verwendeten Begriffe haben grundsätzlich die in der Fachwelt anerkannten Bedeutungen. So sei zu den hier verwendeten Begriffen auf die eingangs zitierte Fachliteratur verwiesen.

Die Dampfreformierung ist beispielsweise bei H.-W. Häring (Hrsg.), "Industrial Gases Processing", Wiley-VCH, 2006, insbesondere Abschnitt 5.2.2.1, "Generation of Synthesis Gas by Steam Reforming", und Abschnitt 5.2.4, "Processes for the Production of Synthesis Gas from Hydrocarbons", beschrieben. Die Autothermreformierung ist entsprechend in Abshnitt 5.2.2.3, "Generation of Synthesis Gas by Autothermal Reforming (ATR)" beschrieben.

Die Dampf- oder Autothermreformierung kann mit methanreichen Einsatzstoffen wie Erdgas betrieben werden. Ausgestaltungen eignen sich auch für die Verarbeitung von leichten, flüssigen Kohlenwasserstoffen wie Naphtha oder Flüssiggas. Für die Dampfreformierung derartiger Einsatzstoffe kann in den Katalysatorrohren ein spezieller Katalysator mit alkalischen Komponenten, meist Pottasche, eingesetzt werden, um eine Rußbildung am Katalysator zu vermeiden. Eine Alternative besteht in der Verwendung einer Vorreformiereinheit (engl. Pre-Reformer), klassischerweise einem adiabatischen Festbettreaktor, der ein vorreformiertes Gemisch umfassend Methan, Kohlenmonoxid und Wasserstoff bei Temperaturen um 450 bis 550 °C erzeugt. Da der Methangehalt bei den genannten Temperaturen noch ausgesprochen hoch ist, wird das erhaltene Gasgemisch anschließend der eigentlichen Dampf- oder Autothermreformierung unterworfen.

Der Begriff "Synthesegas" soll insbesondere ein Wasserstoff und Kohlenmonoxid enthaltendes Gasgemisch bezeichnen, wobei der Gehalt an Wasserstoff und Kohlenmonoxid in Summe insbesondere mehr als 50% auf Gewichtsbasis betragen. Auch Kohlendioxid kann enthalten sein, wobei ein Gehalt an Wasserstoff, Kohlenmonoxid und Kohlendioxid durch eine Wassergaskonvertierung in bekannter Weise beeinflusst werden kann. Ein "Syntheserohgas" enthält insbesondere auch Wasser und nicht umgesetzte Kohlenwasserstoffe des Einsatzes.

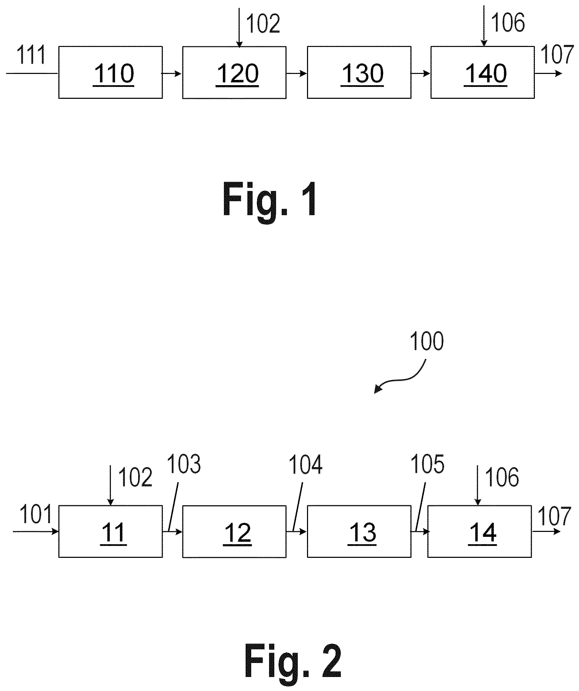

In Figur 1 ist ein nicht erfindungsgemäßes Verfahren in Form eines schematischen Prozessflussdiagramms dargestellt. Das Verfahren gemäß Figur 1 dient insbesondere zur Bearbeitung üblicher Einsätze wie Erdgas, Flüssiggas oder Naphtha, welche keinen besonders erhöhten Gehalt an ungesättigten Kohlenwasserstoffen mit drei und mehr Kohlenstoffatomen aufweisen.

Das in Figur 1 veranschaulichte Verfahren umfasst eine Vorwärmung 110, Hydrierung 120, Entschwefelung 130 und anschließende Reformierung 140 eines entsprechenden Einsatzes 111, wobei die Hydrierung unter Zugabe von Wasserstoff 102 und die Reformierung 140, in Form einer Dampf- oder Autothermreformierung mit optionaler Vorreformierung, unter optionaler Zugabe von Sauerstoff 106 erfolgen kann. Im Ergebnis wird ein Reformierungsprodukt 107 in Form von Syntheserohgas erhalten.

Sollen Einsätze mit einem hohen Anteil an ungesättigten Kohlenwasserstoffen mit drei und mehr Kohlenstoffatomen, wie sie beispielsweise in Ethylenanlagen durch Steamcracken erhalten werden, in Verfahren der in Figur 1 gezeigten Art eingesetzt werden, kann es zu Problemen kommen. So kann es bei der für die Vorreformierung erforderlichen Anwärmung auf die notwendige Temperatur am Eintritt der Vorreformiereinheit zu Polymerisation und/oder Rußbildung kommen.

Ein Einsatz ohne Vorwärmung in einer Partialoxidation ist möglich, jedoch mit den Nachteilen der Partialoxidation gegenüber Verfahren mit Vor- und Dampf- und/oder Autothermreformierung, insbesondere einem hohen Sauerstoffverbrauch, verbunden. Ferner ist bei derartigen Einsätzen die Wärmefreisetzung in der typischerweise bei hohen Temperaturen von 250 bis 400 °C durchgeführten Hydrierung zu hoch, um eine vollständige Hydrierung 120 in einem einzigen Durchlauf realisieren zu können. Ein gekühlter Einsatzgasrecycle wäre erforderlich.

Die nachfolgend nochmals unter Bezugnahme auf den Stand der Technik erläuterten Ausgestaltungen beseitigen diese Nachteile.

Hierbei wird eine Verschaltung einer Flüssighydrierung mit einer Reformierung, insbesondere umfassend eine Vorreformierung sowie eine Dampfreformierung und/oder Autothermreformierung, vorgeschlagen. Dies ermöglicht den Einsatz von längerkettigen, ungesättigten Kohlenwasserstoffen als Einsatzstoff für entsprechende Anlagen, ohne dass aufwendige Maßnahmen zur Kühlung oder Kontrolle der Exothermie in einer konventionellen Hydrierung und Entschwefelung erforderlich werden. Außerdem wird die Gefahr der Rußbildung oder Polymerisation, wie sie bei Anwärmung längerkettiger ungesättigter Kohlenwasserstoffen zu erwarten ist, in entsprechenden Ausgestaltungen umgangen.

In Figur 2 ist ein Verfahren gemäß einer vorgeschlagenen Ausgestaltung in Form eines schematischen Prozessflussdiagramms dargestellt und mit 100 bezeichnet.

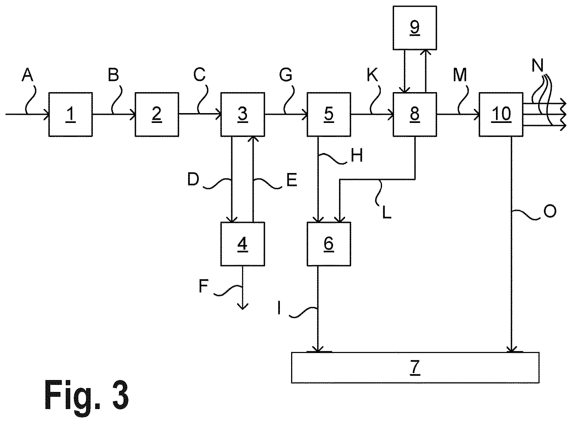

Das Verfahren 100 umfasst ein Bereitstellen eines flüssigen Hydrierungseinsatzes 101 mit ungesättigten Kohlenwasserstoffen mit drei und mehr Kohlenstoffatomen wie anhand eines Beispiels in Figur 3 erläutert, ein Hydrieren 11 des Hydrierungseinsatzes 101 oder eines Teils hiervon in flüssigem Zustand mit Wasserstoff 102 unter Erhalt eines Hydrierungsprodukts 103, ein Aufbereiten 12, 13 des Hydrierungsprodukts 103 oder eines Teils hiervon unter Erhalt eines Reformierungseinsatzes 105, ggf. umfassend die Bildung eines Zwischenprodukts 104, und ein Reformieren 14 des Reformierungseinsatzes 105 mit optionaler Zugabe von Wasserstoff 106 unter Erhalt eines Reformierungsprodukts 107. Die Reformierung 14 kann auch hier eine Vorreformierung und eine Dampf- und/oder Autothermreformierung umfassen. Das Aufbereiten 12, 13 des Hydrierungsprodukts 103 kann insbesondere, und falls erforderlich, eine Vorwärmung 12 und eine Entschwefelung 13 bei Bedarf umfassen.

Es versteht sich, dass das Verfahren 100 auch eine weitere Aufbereitung des Reformierungsprodukts 107, das zu Anfang als Syntheserohgas erhalten wird, umfassen kann, beispielsweise eine Kühlung, Verdichtung, Trocknung, Fraktionierung, Wassergaskonversion und dergleichen, und dass Komponenten wie Kohlenmonoxid, Kohlendioxid und/oder Wasserstoff zu weiteren Verfahrensprodukten wie Ammoniak, Methanol, Ameisensäure und dergleichen umgesetzt werden können.

In Figur 3 ist eine Bereitstellung des Hydriereinsatzes 1, wie sie in einer hier vorgeschlagenen Ausgestaltung vorgenommen werden kann, in Form eines schematischen Prozessflussdiagramms veranschaulicht. Die hier vorgeschlagenen Ausgestaltungen können dabei mit den in Figur 3 veranschaulichten Verfahrensschritten, aber auch mit beliebigen anderen Verfahrensschritten, wie sie beim zum Steamcracken typisch sind, realisiert werden. Es versteht sich, dass nicht alle in Figur 3 veranschaulichten Verfahrensschritte realisiert sein müssen.

Gemäß Figur 3 werden ein oder mehrere Kohlenwasserstoffe oder Kohlenwasserstoffgemische A zusammen mit Dampf dem Steamcracken 1 unterworfen. Die Kohlenwasserstoffe werden zumindest teilweise thermisch gespalten. Zum Steamcracken 1 können ein oder mehrere gleiche oder unterschiedliche Cracker oder Spaltöfen bekannter Art verwendet werden.

Durch das Steamcracken 1 wird ein Komponentengemisch oder Spaltgas B erhalten, welches einem Quench 2 unterworfen wird. Nach diesem wird das Komponentengemisch, nun mit C bezeichnet, einer Ölentfernung 3 zugeführt. In der Ölentfernung 3 wird, falls in dem Komponentengemisch C enthalten, Pyrolyseöl D in einer oder mehreren Fraktionen aus dem Komponentengemisch C abgetrennt. Das Pyrolyseöl D wird im dargestellten Beispiel einer Ölstrippung 4 unterworfen, um mit dem Pyrolyseöl D abgeschiedene leichtere Verbindungen E zurückzugewinnen. Diese werden in die Ölentfernung 3 zurückgeführt. Der verbleibende Rest F des Pyrolyseöls D kann als Rücklauf in die Ölentfernung 3 zurückgeführt und als Produkt in Form von Cracköl bereitgestellt werden. Zusätzlich oder alternativ kann auch nicht der Ölstrippung 4 unterworfenes Pyrolyseöl D als Rücklauf in die Ölentfernung 3 zurückgeführt werden.

Ein nach der Ölentfernung 3 verbleibender Rest G des Komponentengemischs C oder das gesamte Komponentengemisch C, falls keine Ölentfernung 3 erfolgt, wird einer Benzinentfernung 5 zugeführt, deren Vorhandensein und Ausgestaltung sich nach dem Gehalt von Pyrolysebenzin in dem Komponentengemisch C richtet. In der Benzinentfernung 5 wird schweres Pyrolysebenzin H abgetrennt. Das schwere Pyrolysebenzin H wird im dargestellten Beispiel zumindest teilweise einer Benzinstrippung 6 zugeführt, um leichte Komponenten zu entfernen. Letztere können ausgeleitet oder an geeigneter Stelle zurückgeführt werden. Ein Teil des schweren Pyrolysebenzins H kann vor und/oder nach der Benzinstrippung 6 in die Ölentfernung 3 zurückgeführt werden. Das in der Benzinstrippung 6 erhaltene gestrippte Pyrolysebenzin, nun mit I bezeichnet, wird dem sogenannten Benzinweg 7 zugeführt, der hier nicht näher erläutert wird. Es kann auch vorgesehen sein, einen Teil des schweren Pyrolysebenzins H ohne Strippung direkt dem Benzinweg 7 zuzuführen.

Ein nach der Benzinentfernung 5 verbleibender Rest K des Komponentengemischs G oder auch das gesamte Komponentengemisch G, falls keine Benzinentfernung 5 vorgesehen ist, wird im dargestellten Beispiel einer insbesondere mehrstufigen Verdichtung 8, der sogenannten Rohgasverdichtung, zugeführt, im Zuge derer eine Sauergasentfernung 9 erfolgen kann. In der Rohgasverdichtung 6 kann weiteres Pyrolysebenzin L abgeschieden werden, das beispielsweise ebenfalls der Benzinstrippung 6 oder direkt dem Benzinweg 7 zugeführt werden kann.

Das von Sauergasen befreite, verdichtete Komponentengemisch M wird einer Fraktionierung 10 zugeführt, in der mehrere Fraktionen, hier exemplarisch mit N veranschaulicht, gebildet werden. Die Fraktionierung kann unter Verwendung beliebiger Apparate durchgeführt werden. Die Fraktionen N umfassen beispielsweise Fraktionen, die überwiegend oder ausschließlich Verbindungen mit zwei, drei, vier oder mehr als vier Kohlenstoffatomen aufweisen, oder entsprechende Sammelfraktionen oder spezifische Kohlenwasserstoffe wie Ethan oder Ethylen. Die Fraktionen N werden einer geeigneten Verwendung zugeführt. Ihre Bildung richtet sich wiederum nach den dem Steamcracken 1 unterworfenen Kohlenwasserstoffe und damit den entsprechenden Gehalten in dem Komponentengemisch B. In der Fraktionierung 10 kann weiteres Pyrolysebenzin O gebildet werden, das jedoch vorteilhafterweise nicht der Benzinstrippung 6 zugeführt wird. Das Pyrolysebenzin kann dem Benzinweg 7 beispielsweise an anderer Stelle zugeführt werden.

Eine der Fraktionen N kann im Wesentlichen ungesättigte Kohlenwasserstoffe mit drei und mehr Kohlenstoffatomen aufweisen und, ggf. nach weiterer Aufbereitung, als Hydrierungseinsatz 1 in einem in Figur 2 veranschaulichten Verfahren 100 verwendet werden. Die in Figur 3 veranschaulichten Verfahrensschritte können dabei Teil eines hier vorgeschlagenen Verfahrens 100 sein, das damit eine Vielzahl von Verfahrensprodukten bereitstellen kann.

## Patentansprüche

1. Verfahren (100) zur Erzeugung eines oder mehrerer Verfahrensprodukte, wobei das Verfahren (100) umfasst:
Bereitstellen eines flüssigen Hydrierungseinsatzes (101), der ungesättigte Kohlenwasserstoffe mit drei und mehr Kohlenstoffatomen enthält;
Hydrieren (11) des Hydrierungseinsatzes (101) oder eines Teils hiervon in flüssigem Zustand unter Erhalt eines Hydrierungsprodukts (103);
Bilden (12, 13) eines Reformierungseinsatzes (105) unter Verwendung des Hydrierungsprodukts (103) oder eines Teils hiervon; und
Reformieren (14) des Reformierungseinsatzes (105) unter Erhalt eines Reformierungsprodukts (107).

2. Verfahren (100) nach Anspruch 1, wobei das Bereitstellen des flüssigen Hydrierungseinsatzes (101) das Steamcracken (1) eines Kohlenwasserstoffgemischs (A) unter Erhalt eines Spaltgases (B) und das Bilden des Hydrierungseinsatzes (1) unter Verwendung eines Teils des Spaltgases (B) umfasst.

3. Verfahren (100) nach Anspruch 2, wobei das Bilden des Hydrierungseinsatzes (1) unter Verwendung eines Teils des Spaltgases (B) eine Deethanisierung umfasst.

4. Verfahren (100) nach einem der vorstehenden Ansprüche, wobei das Verfahren (100) eine Bereitstellung einer oder mehrerer Komponenten des Reformierungsprodukts (7) als das Verfahrensprodukt oder eines der mehreren Verfahrensprodukte umfasst.

5. Verfahren (100) nach einem der vorstehenden Ansprüche, wobei das Verfahren (100) eine Umsetzung einer oder mehrerer Komponenten des Reformierungsprodukts (7) zu dem Verfahrensprodukt oder einem der mehreren Verfahrensprodukte umfasst.

6. Verfahren (100) nach Anspruch 5, wobei die Umsetzung eine Umsetzung von Wasserstoff mit Stickstoff zu Ammoniak und/oder von Kohlendioxid mit Wasserstoff zu Methanol umfasst.

7. Verfahren nach einem der vorstehenden Ansprüche, wobei die ungesättigten Kohlenwasserstoffe mit drei und mehr Kohlenstoffatomen mit bis zu 100 Gewichtsprozent in dem Hydrierungseinsatz (101) enthalten sind.

8. Verfahren nach einem der vorstehenden Ansprüche, wobei die Kohlenwasserstoffe mit drei und mehr Kohlenstoffatomen Kohlenwasserstoffe mit bis zu 12 Kohlenstoffatomen umfassen.

9. Verfahren nach einem der vorstehenden Ansprüche, wobei das Reformieren (14) des Reformierungseinsatzes (105) eine Dampf- und/oder Autothermreformierung umfasst.

10. Verfahren nach Anspruch 9, wobei das Reformieren (14) des Reformierungseinsatzes (105) eine Vorreformierung umfasst.

11. Anlage zur Erzeugung eines oder mehrerer Verfahrensprodukte, wobei die Anlage zur Durchführung der folgenden Schritte eingerichtet ist:
Bereitstellen eines flüssigen Hydrierungseinsatzes (101) mit ungesättigten Kohlenwasserstoffen mit drei und mehr Kohlenstoffatomen;
Hydrieren (11) des Hydrierungseinsatzes (101) oder eines Teils hiervon in flüssigem Zustand unter Erhalt eines Hydrierungsprodukts (103);
Bilden (12, 13) eines Reformierungseinsatzes (105) unter Verwendung des Hydrierungsprodukts (103) oder eines Teils hiervon; und
Reformieren (14) des Reformierungseinsatzes (105) unter Erhalt eines Reformierungsprodukts (107).

12. Anlage nach Anspruch 11, wobei die Anlage zur Durchführung eines Verfahrens nach einem der Ansprüche 1 bis 10 eingerichtet ist.
